# EUROPEAN PATENT APPLICATION

(11) **EP 4 789 709 A1**
(43) Date of publication of application: **12.08.2026**
(21) Application number: 24874428.6
(22) Date of filing: 09.09.2024
(51) Int. Cl.: A61M 25/09

(54) **GUIDE WIRE**

(30) Priority: 04.10.2023 JP 2023172503
(71) Applicant: Asahi Intecc Co., Ltd., Seto-shi, Aichi 489-0071 (JP)
(72) Inventor: FUJIWARA, Soichiro, Seto-shi, Aichi 489-0071 (JP); NISHIGISHI, Makoto, Seto-shi, Aichi 489-0071 (JP); SUGITA, Rio, Seto-shi, Aichi 489-0071 (JP)
(74) Representative: Prinz & Partner mbB
(86) International application number: PCT/JP2024/032217
(87) International publication number: WO 2025/074816

(57) **Abstract**

A guide wire 1 includes a core shaft 11, a coil body 21 in which a wire w is wound around so as to cover an outer periphery of a distal end portion of the core shaft 11, a first portion 41 including a hydrophilic coating 411 disposed on a surface located on a radially outward side among outer peripheral surfaces of the wire w and a hydrophobic coating 412 formed on the hydrophilic coating 411, and a second portion 51 including a hydrophilic coating 511, the second portion 51 being a portion on a radially outward side of an axial center of the wire w so as to fill concave portions between wires or turns of the wire w, w adjacent to each other along a longitudinal direction.

## Description

### TECHNICAL FIELD

The present invention relates to a guide wire.

### BACKGROUND ART

For example, when a therapeutic instrument (accompanying device) such as a catheter is inserted into a body cavity such as a blood vessel, a guide wire is inserted into the body cavity in advance to guide the accompanying device to a site to be treated.

A guide wire is inserted into a body cavity after, for example, shaping a distal end portion into a substantially J-shape in advance, and at a location where the body cavity branches, the guide wire is appropriately rotated so that the distal end portion of the guide wire faces a desired branching vessel, and the distal end portion is pushed forward to the site to be treated.

For such a guide wire, in order to reach the site to be treated quickly, techniques have been proposed for reducing frictional resistance with an accompanying device by, for example, reducing a contact area of the guide wire in contact with a wall surface (see, for example, Patent Literature 1), or providing a hydrophilic coating on a surface of the guide wire in contact with the wall surface to form a liquid film on the outermost surface while absorbing moisture upon contact with body fluid (see, for example, Patent Literature 2).

### CITATION LIST

### Patent Literature

Patent Literature 1: JP 2011-152211 A
Patent Literature 2: JP 2012-70979 A

### SUMMARY OF INVENTION

### TECHNICAL PROBLEM

However, even when a liquid film is formed on the outermost surface using a hydrophilic coating, if the hydrophilic coating swells excessively due to moisture absorption, for example, a clearance with an accompanying device contacting an outer peripheral surface becomes small. For this reason, frictional resistance with the accompanying device increases on the contrary, and when rotating the distal end portion of the guide wire by a rotation operation at hand, a rotation response of the guide wire may deteriorate.

The present invention has been made based on the above circumstances, and an object thereof is to provide a guide wire capable of improving a rotation response of the guide wire while suppressing an increase in frictional resistance with an accompanying device due to swelling of a hydrophilic coating, even when the hydrophilic coating comes into contact with a liquid such as body fluid.

### SOLUTION TO PROBLEM

Some aspects of the present disclosure are:
(1) a guide wire including: a core shaft; a coil body in which a wire or wires are wound around so as to cover an outer periphery of a distal end portion of the core shaft; a first portion including a hydrophilic coating disposed on a surface located on a radially outward side among outer peripheral surfaces of the wire or wires, and a hydrophobic coating formed on an outer peripheral surface of the hydrophilic coating; and a second portion including a hydrophilic coating, the second portion being a portion on a radially outward side of an axial center of the wire or wires so as to fill concave portions between wires or turns of the wire adjacent to each other along a longitudinal direction;
(2) the guide wire according to (1), wherein when the hydrophilic coating is dry, a distance between an outer peripheral surface of the second portion and a center axis of the core shaft is smaller than a distance between an outer peripheral surface of the first portion and the center axis of the core shaft;
(3) the guide wire according to (1), wherein when the hydrophilic coating is in contact with a liquid, the distance between the outer peripheral surface of the second portion and the center axis of the core shaft is substantially equal to the distance between the outer peripheral surface of the first portion and the center axis of the core shaft; and
(4) a guide wire including: a core shaft; a coil body in which a wire or wires are wound around so as to cover an outer periphery of a distal end portion of the core shaft; a first portion including a hydrophobic coating disposed on a surface located on a radially outward side among outer peripheral surfaces of the wire or wires; and a second portion including a hydrophilic coating, the second portion being a portion on a radially outward side of an axial center of the wire so as to fill concave portions between wires or turns of the wire adjacent to each other along a longitudinal direction.

As used herein, "distal end side" means a direction along the longitudinal direction of the guide wire and a direction to be inserted into a deeper part (distal) of a body cavity. "Proximal end side" means a direction along the longitudinal direction of the guide wire and a direction opposite to the "distal end side." "Distal end" indicates a distal end side end in an arbitrary member or portion, and "proximal end" indicates a proximal end side end in an arbitrary member or portion, respectively. "Distal end portion" refers to a portion in an arbitrary member or portion that includes the distal end thereof and extends from this distal end toward the proximal end side to the middle in the longitudinal direction. "Proximal end portion" refers to a portion in an arbitrary member or portion that includes the proximal end thereof and extends from this proximal end toward the distal end side to the middle in the longitudinal direction. "Longitudinal direction" refers to the longitudinal direction of the core shaft unless otherwise specified. "Radial direction" refers to a radial direction orthogonal to the longitudinal direction of the core shaft.

### ADVANTAGEOUS EFFECTS OF INVENTION

According to the present invention, even when the hydrophilic coating comes into contact with a liquid such as body fluid, it is possible to provide a guide wire capable of improving the rotation response of the guide wire while suppressing an increase in frictional resistance with an accompanying device due to swelling of the hydrophilic coating.

### BRIEF DESCRIPTION OF DRAWINGS

FIG. 1 is a schematic longitudinal sectional view showing a first embodiment.
FIG. 2 is a schematic side view showing a part of the first embodiment in an enlarged manner.
FIG. 3 is a schematic longitudinal sectional view showing a part of the first embodiment in an enlarged manner, in a state where a hydrophilic coating is dry.
FIG. 4 is a schematic longitudinal sectional view showing a part of the first embodiment in an enlarged manner, in a state where the hydrophilic coating is in contact with a liquid.
FIG. 5A is a schematic sectional view showing an example of a state of the guide wire when the hydrophilic coating is dry in the first embodiment.
FIG. 5B is a schematic sectional view showing an example of a state of the guide wire when the hydrophilic coating is in contact with a liquid in the first embodiment.
FIG. 6 is a schematic longitudinal sectional view showing a part of a second embodiment in an enlarged manner, in a state where a hydrophilic coating is dry.
FIG. 7 is a schematic longitudinal sectional view showing a part of the second embodiment in an enlarged manner, in a state where the hydrophilic coating is in contact with a liquid.
FIG. 8A is a schematic longitudinal sectional view showing a modification of a second portion.
FIG. 8B is a schematic longitudinal sectional view showing a modification of the second portion.
FIG. 8C is a schematic longitudinal sectional view showing a modification of the second portion.

### DESCRIPTION OF EMBODIMENTS

A guide wire of the present disclosure includes a core shaft, a coil body in which a wire or wires are wound around so as to cover an outer periphery of a distal end portion of the core shaft, a first portion including a hydrophilic coating disposed on a surface located on a radially outward side among outer peripheral surfaces of the wire or wires and a hydrophobic coating formed on an outer peripheral surface of the hydrophilic coating, and a second portion including a hydrophilic coating, the second portion being a portion on a radially outward side of an axial center of the wire or wires so as to fill concave portions between wires or turns of the wire adjacent to each other along a longitudinal direction.

The present disclosure includes a guide wire including a core shaft, a coil body in which a wire or wires are wound around so as to cover an outer periphery of a distal end portion of the core shaft, a first portion including a hydrophobic coating disposed on a surface located on a radially outward side among outer peripheral surfaces of the wire or wires, and a second portion consisting of a hydrophilic coating, the second portion being a portion on a radially outward side of an axial center of the wire or wires so as to fill concave portions between wires or turns of the wire adjacent to each other along a longitudinal direction.

The rotation response means the easiness of starting the rotation behavior of the guide wire distal end portion in response to a rotation operation of the guide wire proximal end portion, and torquability means the ability to transmit the rotational force applied to the guide wire proximal end portion to the guide wire distal end portion.

Hereinafter, first and second embodiments of the disclosed embodiments will be described with reference to the drawings, but the disclosed embodiments are not limited only to the embodiments described in the drawings. Also, dimensions of each part shown in the drawings are dimensions shown for facilitating understanding of the implementation details, and do not necessarily correspond to actual dimensions.

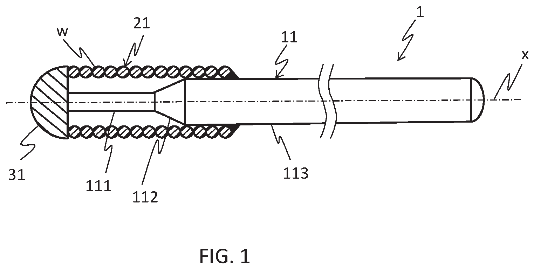

In FIGS. 1 to 8C, the left side in the drawings is the distal end side (distal side) to be inserted into a deeper part (distal) of a body cavity, and the right side in the drawings is the proximal end side (hand side, near side).

### [First Embodiment]

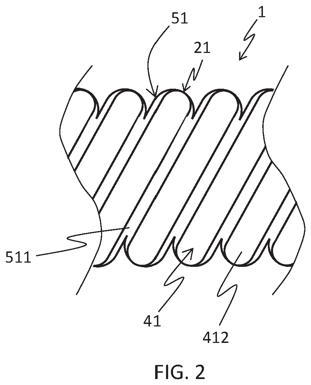

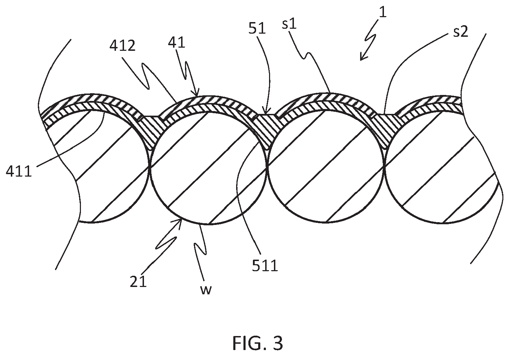

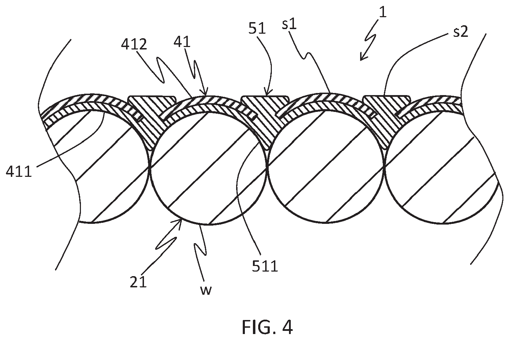

FIGS. 1 to 4 are schematic views showing the first embodiment. As shown in FIGS. 1 and 2, a guide wire 1 is schematically configured by a core shaft 11, a coil body 21, a distal end fixing portion 31, a first portion 41, and a second portion 51. FIG. 3 shows a state in which the guide wire 1 is not in contact with a liquid (a state when hydrophilic coatings 411 and 511 are dry), and FIG. 4 shows a state in which the guide wire 1 is in contact with a liquid (a state when the hydrophilic coatings 411 and 511 are in contact with a liquid).

The core shaft 11 is a longitudinal member constituting an axial core of the guide wire 1. The core shaft 11 can be formed of, for example, a flexible material, and the distal end portion thereof can be configured to gradually decrease in diameter toward the distal end side.

In the present embodiment, the distal end portion of the core shaft 11 includes a small diameter portion 111 having a constant outer diameter, a large diameter portion 113 located on the proximal end side of the small diameter portion 111 and having a constant outer diameter larger than that of the small diameter portion 111, and a tapered portion 112 disposed so as to be continuous with the small diameter portion 111 and the large diameter portion 113 and gradually increasing in diameter from the small diameter portion 111 toward the large diameter portion 113. That is, in a state where the core shaft 11 extends linearly, the small diameter portion 111 and the large diameter portion 113 each have a cylindrical shape with a constant outer diameter, and the tapered portion 112 has a truncated conical shape gradually increasing in diameter toward the proximal end side.

Although not illustrated, the core shaft 11 may further have a truncated conical connection portion whose distal end is continuous with the proximal end of the large diameter portion 113, a cylindrical main body part whose distal end is continuous with the proximal end of the connection portion and has a constant outer diameter, and the like.

As a material constituting the core shaft 11, it is preferable to ensure flexibility and have anti-thrombogenicity and biocompatibility. Examples of the material include stainless steel such as SUS304, a superelastic alloy such as an Ni-Ti alloy, and the like.

The coil body 21 is a spiral (coil-shaped) member in which a wire or wires are wound around so as to cover the outer periphery of the distal end portion of the core shaft 11. Specifically, the coil body 21 can be formed by winding a wire w in a single thread or multi-threads so as to cover at least a part of the core shaft 11. The coil body 21 may be loosely wound having gaps between wires or turns of the wire along the longitudinal direction, or may be closely wound in which wires or the turns of the wire are in contact with each other.

As the wire w constituting the coil body 21, one or a plurality of solid wires, or one or a plurality of twisted wires can be used. However, the solid wire means a single single-wire, and the twisted wire means a bundle of wires formed by twisting a plurality of single-wires together in advance.

As a wire material constituting the coil body 21, from the viewpoint of ensuring flexibility of the guide wire 1 and imparting anti-thrombogenicity and biocompatibility, for example, stainless steel such as SUS316; a superelastic alloy such as an Ni-Ti alloy; a radiopaque metal such as platinum or tungsten, and the like can be adopted.

The distal end fixing portion 31 is a portion where the distal end of the core shaft 11 and the distal end of the coil body 21 are integrally fixed. Specifically, the distal end fixing portion 31 can be formed to have, for example, a substantially hemispherical shape in which a distal end portion is curved in a convex shape toward the distal end side. This makes it possible to reduce resistance when the guide wire 1 advances in a body cavity, and the guide wire 1 can be smoothly inserted.

As a method for forming the distal end fixing portion 31, for example, a method for forming by melt-molding a part of the members constituting the core shaft 11 and/or the coil body 21, a method for joining the core shaft 11 and the coil body 21 using a brazing material and forming by molding this brazing material, and the like can be adopted. Examples of the brazing material include metal brazing materials such as an Sn-Pb alloy, a Pb-Ag alloy, an Sn-Ag alloy, and an Au-Sn alloy.

The proximal end portion of the coil body 21 can be joined to the core shaft 11. Specifically, for example, the proximal end portion of the coil body 21 may be joined to the outer peripheral surface of the core shaft 11 (for example, the outer peripheral surface of the large diameter portion 113 and the like) using a brazing material. Examples of the brazing material for joining the proximal end portion of the coil body 21 include a brazing material similar to the brazing material that formed the distal end fixing portion.

The first portion 41 is a portion including a hydrophilic coating disposed on a surface located on the radially outward side among the outer peripheral surfaces of the wire w and a hydrophobic coating formed on the outer peripheral surface of the hydrophilic coating. Specifically, as shown in FIGS. 2 and 3, the first portion 41 is provided on the outer peripheral surface side of the coil body 21, that is, on the surface of the wire w located on the radially outward side from the center of the wire w. The first portion 41 is laminated in the order of the hydrophilic coating 411 and the hydrophobic coating 412 from the wire w side. Note that the static friction coefficient of each surface when in contact with a liquid increases in the order of hydrophilic coating, hydrophobic coating, and wire.

The hydrophilic coating 411 improves slidability (reduces frictional resistance) between the surface of the guide wire 1 and a wall surface of a body cavity, or between the surface of the guide wire 1 and an accompanying device (not illustrated) through which the guide wire 1 is inserted, for example, when the guide wire 1 is inserted into a body cavity.

Examples of the material constituting the hydrophilic coating 411 include polyvinyl alcohol, polyvinylpyrrolidone, polyethylene glycol, polyacrylamide, polyacrylic acid, sodium polyacrylate, poly(2-hydroxyethyl methacrylate), maleic anhydride-based copolymers, ethylene vinyl alcohol copolymers, 2-methacryloyloxyethyl phosphorylcholine or copolymers thereof, (2-hydroxyethyl methacrylate)-styrene block copolymers, various synthetic polypeptides, collagen, hyaluronic acid, cellulosic polymers, and mixtures thereof. Note that the hydrophilic coating 411 may contain additives such as a crosslinking agent, a non-volatile solvent, a volatile solvent, and a surfactant.

The hydrophobic coating 412 limits, for example, the amount of liquid penetrating into the deep part of the first portion 41. This hydrophobic coating 412 can suppress the liquid from reaching the hydrophilic coating 411 located in the deep part of the first portion 41, and can reduce excessive swelling of the hydrophilic coating 411 due to the penetrated liquid.

Examples of the material constituting the hydrophobic coating 412 include silicone, polytetrafluoroethylene (PTFE), fluorinated ethylene propylene (FEP), and mixtures thereof. Note that the hydrophobic coating 412 may contain additives such as a crosslinking agent, a non-volatile solvent, a volatile solvent, and a surfactant.

The second portion 51 is a portion on the radially outward side of the axial center of the wire w, and is a portion including a hydrophilic coating disposed so as to fill concave portions between wires or adjacent turns of the wire along the longitudinal direction. Specifically, as shown in FIGS. 2 and 3, the second portion 51 is disposed on the outer peripheral surface side of the coil body 21, that is, so as to fill concave portions (valley) generated between wires or adjacent turns of the wire w, w located on the radially outward side from the axial center of the wire w.

The hydrophilic coating 511 in the second portion 51 also improves slidability (reduces frictional resistance) between the surface of the guide wire 1 and the wall surface of the body cavity, or between the surface of the guide wire 1 and an accompanying device through which the guide wire 1 is inserted, for example, when the guide wire 1 is inserted into a body cavity.

Examples of the material constituting the hydrophilic coating 511 in the second portion 51 include those similar to the material of the hydrophilic coating 411 described above. The material constituting the hydrophilic coating 511 may be the same as or different from the material constituting the hydrophilic coating 411.

Here, states of the first and second portions 41 and 51 when the guide wire 1 is not in contact with a liquid (a state when the hydrophilic coatings 411 and 511 are dry), and states of the first and second portions 41 and 51 when the guide wire 1 is in contact with a liquid (a state when the hydrophilic coatings 411 and 511 are in contact with a liquid) will be described.

FIG. 3 is a schematic view when the hydrophilic coatings 411 and 511 are dry, and FIG. 4 is a schematic view when the hydrophilic coatings 411 and 511 are in contact with a liquid. For example, when the guide wire 1 is inserted into a body cavity and comes into contact with a surrounding liquid such as body fluid, in the first portion 41, the liquid penetrates into the hydrophilic coating 411 through the hydrophobic coating 412 located on the surface. When the liquid penetrates to the hydrophilic coating 411, the hydrophilic coating 411 takes in the liquid, and a liquid film (not illustrated) is formed on the surface side (radially outward side) of the hydrophilic coating 411. The formed liquid film expands in volume by the taken-in liquid and exhibits viscosity.

At this time, if there is no hydrophobic coating and the hydrophilic coating is exposed on the surface, the clearance between the guide wire and an inner wall of an accompanying device through which the guide wire is inserted becomes small due to the hydrophilic coating swollen by taking in body fluid. For this reason, even if a liquid film is formed, frictional resistance between the guide wire and the accompanying device increases on the contrary, and smooth operation of the accompanying device is hindered.

However, in the present embodiment, as shown in FIG. 4, since the hydrophobic coating 412 is laminated on the hydrophilic coating 411 in the first portion 41, this hydrophobic coating 412 limits the amount of liquid penetrating into the hydrophilic coating 411 located in the deep part of the first portion 41. As a result, swelling of the hydrophilic coating 411 is suppressed, and by ensuring the clearance between the guide wire 1 and the inner wall of the accompanying device, frictional resistance between the guide wire 1 and the accompanying device can be reduced.

On the other hand, in the second portion 51, there is no hydrophobic coating, and the hydrophilic coating 511 is exposed on the surface. For this reason, when the guide wire 1 comes into contact with a liquid, since there is no hydrophobic coating on the surface, more liquid penetrates into the hydrophilic coating 511, and the hydrophilic coating 511 swells according to the amount of liquid taken in. As a result, the outer peripheral surface s2 of the second portion 51 located in the valley between wires or the adjacent turns of the wire w bulges toward the radially outward side, and frictional resistance between the guide wire 1 and the accompanying device is reduced by the liquid film on the bulged surface.

The ratio of the areas of the first portion 41 and the second portion 51 on the outermost surface of the guide wire 1 during drying is not particularly limited. The ratio of the surface area of the first portion 41 to the surface area of the second portion 51 may be set to, for example, 1:10 to 10:1.

The thicknesses of the hydrophilic coating 411 and the hydrophobic coating 412 of the first portion 41 during drying are not particularly limited as long as the effect of the present invention is not impaired. The thickness of each of the hydrophilic coating 411 and the hydrophobic coating 412 of the first portion 41 may be set to, for example, 1 µm to 300 µm.

The radial thickness of the hydrophilic coating 511 of the second portion 51 during drying is not particularly limited as long as the effect of the present invention is not impaired. The thickness of the hydrophilic coating 511 of the second portion 51 may be set to, for example, 1 µm to 300 µm.

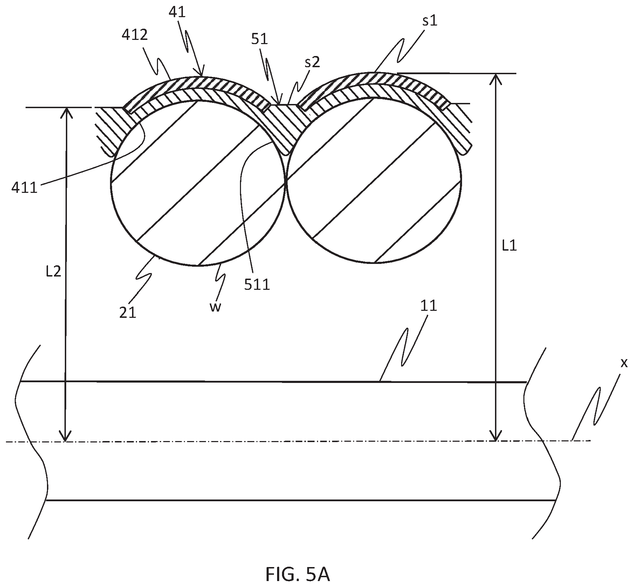

Note that when the hydrophilic coatings 411 and 511 are dry, a distance L2 between the outer peripheral surface s2 of the second portion 51 and the center axis x of the core shaft 11 is preferably smaller than a distance L1 between an outer peripheral surface s1 of the first portion 41 and the center axis x of the core shaft 11, as shown in FIG. 5A. Accordingly, even when the hydrophilic coating 511 of the second portion 51 is in contact with a liquid, the second portion 51 can be suppressed from protruding further toward the radially outward side than the first portion 41, and coupled with the formation of the liquid film, frictional resistance with an accompanying device contacting the second portion 51 can be further reduced.

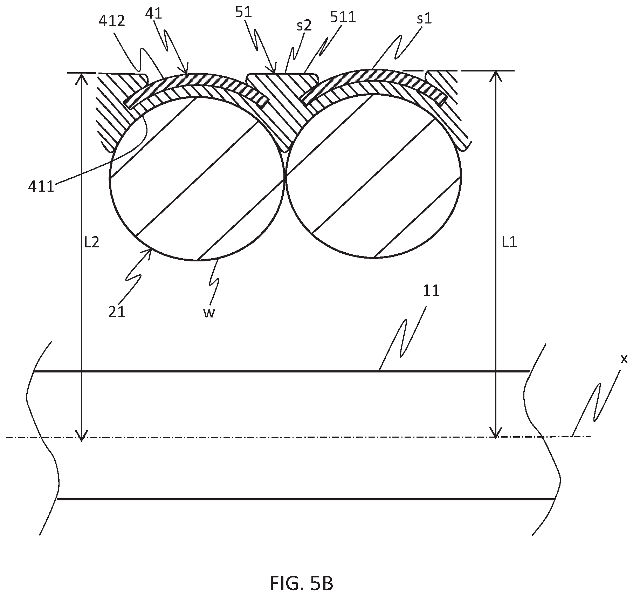

Further, when the hydrophilic coatings 411 and 511 are in contact with a liquid, as shown in FIG. 5B, it is also preferable that the distance L2 between the outer peripheral surface s2 of the second portion 51 and the center axis x of the core shaft 11 is substantially equal to the distance L1 between the outer peripheral surface s1 of the first portion 41 and the center axis x of the core shaft 11. Accordingly, when the hydrophilic coating 511 of the second portion 51 is in contact with a liquid, since the difference in unevenness between the outer peripheral surfaces s1 and s2 of the first and second portions 41 and 51 is small, slidability and the like with an accompanying device can be improved.

Next, a usage mode of the guide wire 1 will be described. Here, a procedure for inserting the guide wire 1 into a blood vessel is exemplified.

First, in order to improve blood vessel selectivity by a rotation operation and the like, before inserting the guide wire 1 into a blood vessel, the distal end portion thereof is curved into a J-shape or the like in advance as necessary.

Next, the guide wire 1 is inserted into a blood vessel from its distal end, and the distal end portion is pushed forward by operating the proximal end portion of the guide wire 1 exposed outside the body. Note that at a branching part of a blood vessel, a rotation operation is applied to the guide wire 1 so that the distal end portion of the guide wire 1 faces a desired blood vessel. At this time, the guide wire 1 can be easily rotated due to excellent rotation response.

Next, after the distal end portion of the guide wire 1 reaches a treatment site, the proximal end of the guide wire 1 is inserted into a lumen of an accompanying device (not illustrated) such as a catheter from its distal end, and the accompanying device is pushed forward in the blood vessel along the guide wire 1. Then, after the accompanying device reaches the treatment site, various treatments are performed using the accompanying device. Then, after the treatments are completed, a series of procedures is ended by pulling out the accompanying device and the guide wire 1 from the blood vessel.

As described above, since the guide wire 1 has the above configuration, even when the hydrophilic coating 411 comes into contact with a liquid such as body fluid, the rotation response of the guide wire 1 can be improved while suppressing an increase in frictional resistance (particularly, static friction coefficient) with an accompanying device due to swelling of the hydrophilic coating 411. In addition, since the frictional resistance with an accompanying device is improved, it is also possible to improve torquability.

The reason why rotation response and torquability are improved is presumed to be as follows. That is, in the first portion 41, the presence of the hydrophobic coating 412 limits the penetration of body fluid contacting the surface of the first portion 41. Therefore, swelling of the hydrophilic coating 411 located directly under the hydrophobic coating 412 can be suppressed while forming a liquid film covering a part of the outermost surface (the surface in contact with an accompanying device) of the first portion 41 by the body fluid that has moderately penetrated into the hydrophilic coating 411. As a result, it is presumed that an increase in frictional resistance with an accompanying device due to swelling of the hydrophilic coating 411 is suppressed, and coupled with the formation of the liquid film, rotation response and torquability of the guide wire 1 are improved.

Note that, although not shown in FIG. 4, when the first portion 41 is in contact with a liquid, it may be in a state where a part of the hydrophilic coating 411, which was located directly under the hydrophobic coating 412 during drying, exudes onto the hydrophobic coating 412 and a liquid film is formed on the outermost surface (the surface of the guide wire in contact with an accompanying device), or it may be in a state where a liquid film covering a part of the outermost surface is formed while the hydrophilic coating 411 remains directly under the hydrophobic coating 412.

### [Second Embodiment]

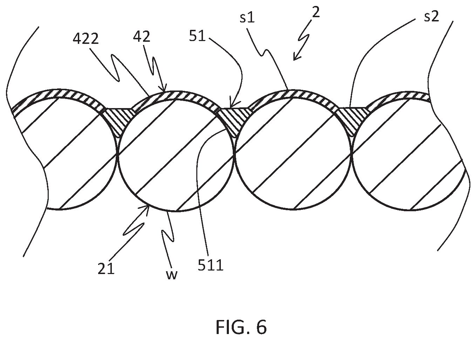

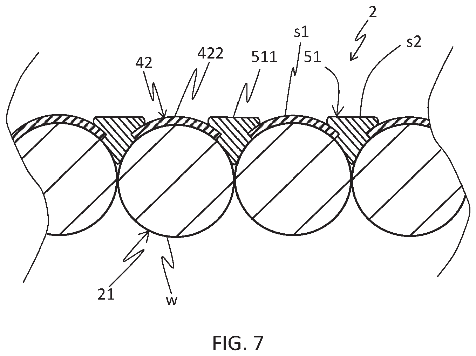

FIGS. 6 and 7 are enlarged schematic longitudinal sectional views showing a part of a second embodiment (a part of a coil body). A guide wire 2 is schematically configured by a first portion 42, and a core shaft 11, a coil body 21, a distal end fixing portion 31, and a second portion 51, which are not shown. FIG. 6 shows a state in which the guide wire 2 is not in contact with a liquid (a state when a hydrophilic coating 511 is dry), and FIG. 7 shows a state in which the guide wire 2 is in contact with a liquid (a state when the hydrophilic coating 511 is in contact with a liquid). The guide wire 2 differs from the first embodiment in the configuration of the first portion 42. Configurations of the core shaft 11, the coil body 21, the distal end fixing portion 31, and the second portion 51, and configurations other than the configuration of the first portion 42 shown below are similar to those of the first embodiment, and therefore the same parts are denoted by the same symbols and detailed description thereof will be omitted. In addition, the usage mode of the guide wire 2 is also exemplified by the same one as in the first embodiment.

The first portion 42 is a portion including a hydrophobic coating disposed on a surface located on the radially outward side among the outer peripheral surfaces of the wire w. Specifically, as shown in FIG. 6, the first portion 42 is provided on the outer peripheral surface side of the coil body 21, that is, on the surface of the wire located on the radially outward side from the center of the wire w.

In the first portion 42 of the present embodiment, only a hydrophobic coating 422 is disposed on the surface on the radially outward side of the wire w. This hydrophobic coating 422 makes frictional resistance with an accompanying device smaller than, for example, a case where the wire w and the accompanying device directly contact each other. That is, the static friction coefficient of each surface when in contact with a liquid satisfies the relationship of hydrophobic coating < wire.

Examples of the material constituting the hydrophobic coating 422 include those similar to the material of the hydrophobic coating 412 exemplified in the first embodiment.

Here, states of the first and second portions 42 and 51 when the guide wire 2 is not in contact with a liquid (a state when the hydrophilic coating 511 is dry), and states of the first and second portions 42 and 51 when the guide wire 2 is in contact with a liquid (a state when the hydrophilic coating 511 is in contact with a liquid) will be described.

For example, when the guide wire 2 is inserted into a body cavity and comes into contact with a surrounding liquid such as body fluid, in the first portion 42, the hydrophobic coating 422 comes into contact with the body fluid. Since this hydrophobic coating 422 hardly absorbs body fluid and swelling of the hydrophobic coating 422 itself is negligibly small, the clearance between the guide wire 2 and the inner wall of the accompanying device is maintained, and an increase in frictional resistance between the guide wire 2 and the accompanying device is suppressed.

On the other hand, in the second portion 51, there is no hydrophobic coating, and the hydrophilic coating 511 is disposed on the surface. For this reason, when the hydrophilic coating 511 of the second portion 51 comes into contact with a liquid, the liquid penetrates into the hydrophilic coating 511 without hindrance, and as shown in FIG. 7, the hydrophilic coating 511 swells by a large amount of the taken-in liquid. As a result, the outer peripheral surface s2 of the second portion 51 including the hydrophilic coating 511 disposed so as to fill the space between wires or the adjacent turns of the wire w along the longitudinal direction bulges toward the radially outward side and the unevenness with the outer peripheral surface s1 decreases, and frictional resistance between the guide wire 2 and the accompanying device is reduced by the liquid film formed on the surface of the second portion 51.

As described above, since the guide wire 2 has the above configuration, even when the hydrophilic coating 511 comes into contact with a liquid such as body fluid, since there is no hydrophilic coating directly under the hydrophobic coating 422, the first portion 42 does not excessively swell, and the rotation response of the guide wire 2 can be improved by the hydrophobic coating 422 having a smaller surface friction coefficient than the wire w. In addition, since the frictional resistance with an accompanying device is improved, it is also possible to improve torquability.

The present disclosure is not limited to the configurations of the embodiments described above, but is indicated by the claims and is intended to include all changes within the meaning and range equivalent to the claims. A part of the configurations of the embodiments described above may be deleted or replaced with other configurations, and other configurations may be added to the configurations of the embodiments described above.

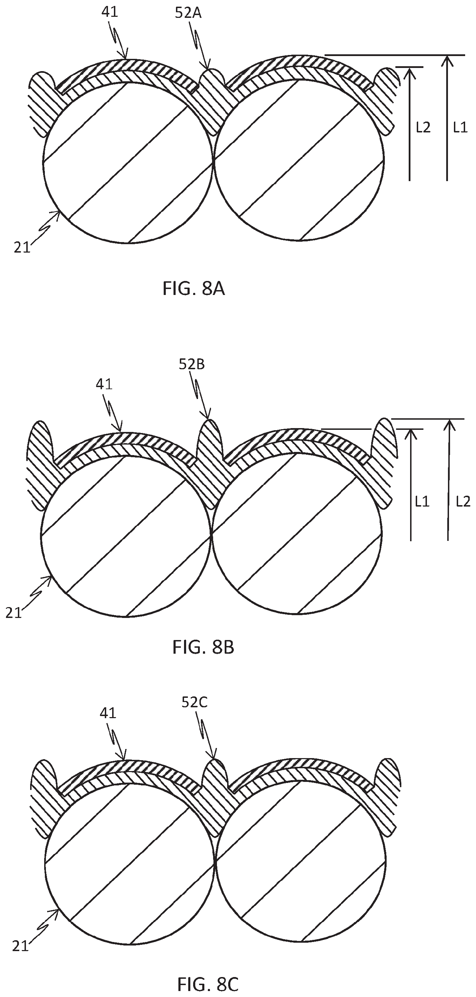

For example, in the above-described embodiment, a configuration has been described in which, when the guide wires 1 and 2 are in contact with a liquid, the distance L2 between the outer peripheral surface s2 of the second portion 51 and the center axis x of the core shaft 11 is substantially equal to the distance L1 between the outer peripheral surface s1 of the first portions 41 and 42 and the center axis x of the core shaft 11. However, the degree to which the second portion swells due to the contacting liquid is not particularly limited. When the guide wire is in contact with a liquid, for example, L2 < L1 may be satisfied (see a second portion 52A in FIG. 8A), or L2 > L1 may be satisfied (see a second portion 52B in FIG. 8B).

Further, the longitudinal sectional shape of the outer peripheral surface of the bulged hydrophilic coating in the second portion when the guide wire is in contact with a liquid is not limited to a substantially linear shape (see FIGS. 4 and 7), and may be, for example, a rounded shape (see a second portion 52C in FIG. 8C).

Further, in the above-described embodiment, the guide wires 1 and 2 in which the hydrophilic coatings 411 and 511 and the hydrophobic coatings 412 and 422 are provided only on the radially outward side among the outer peripheral surfaces of the wire w have been described. However, a hydrophilic coating or a hydrophobic coating may be provided also on a radially inward side among the outer peripheral surfaces of the wire w.

Further, in the above-described embodiment, the guide wires 1 and 2 provided with the coil body 21 in which the wire w or wires are closely wound over the entire longitudinal direction have been described. However, it may be a guide wire provided with a coil body in which at least a part of the longitudinal direction is loosely wound. In the loosely wound coil body, for example, in a sectional view in which the guide wire is cut along the longitudinal direction, wires or adjacent turns of the wire spaced apart from each other may be connected (bridged) via the hydrophilic coating of the second portion.

### REFERENCE SIGNS LIST

1, 2 Guide wire
11 Core shaft
21 Coil body
41, 42 First portion
411 Hydrophilic coating
412, 422 Hydrophobic coating
51 Second portion
511 Hydrophilic coating
L1, L2 Distance
s1, s2 Outer peripheral surface
w Wire

## Claims

1. A guide wire comprising:
a core shaft;
a coil body in which a wire or wires are wound around so as to cover an outer periphery of a distal end portion of the core shaft;
a first portion including of a hydrophilic coating disposed on a surface located on a radially outward side among outer peripheral surfaces of the wire or wires, and a hydrophobic coating formed on an outer peripheral surface of the hydrophilic coating; and
a second portion including a hydrophilic coating, the second portion being a portion on a radially outward side of an axial center of the wire so as to fill concave portions between wires or turns of the wire adjacent to each other along a longitudinal direction.

2. The guide wire according to claim 1, wherein when the hydrophilic coating is dry, a distance between an outer peripheral surface of the second portion and a center axis of the core shaft is smaller than a distance between an outer peripheral surface of the first portion and the center axis of the core shaft.

3. The guide wire according to claim 1, wherein when the hydrophilic coating is in contact with a liquid, a distance between an outer peripheral surface of the second portion and a center axis of the core shaft and a distance between an outer peripheral surface of the first portion and the center axis of the core shaft are substantially equal.

4. A guide wire comprising:
a core shaft;
a coil body in which a wire or wires are wound around so as to cover an outer periphery of a distal end portion of the core shaft;
a first portion including a hydrophobic coating disposed on a surface located on a radially outward side among outer peripheral surfaces of the wire or wires; and
a second portion including a hydrophilic coating, the second portion being a portion on a radially outward side of an axial center of the wire so as to fill concave portions between wires or turns of the wire adjacent to each other along a longitudinal direction.
